(19) Europäisches Patentamt
European Patent Office
Office européen des brevets

(11) **EP 3 286 251 B1**

(12) **EUROPEAN PATENT SPECIFICATION**

(45) Date of publication and mention
of the grant of the patent:
**20.02.2019 Bulletin 2019/08**

(51) Int Cl.:
**C08J 3/09** *(2006.01)*  **C08J 7/02** *(2006.01)*
**C08J 3/02** *(2006.01)*

(21) Application number: **16723192.7**

(22) Date of filing: **13.04.2016**

(86) International application number:
**PCT/US2016/027294**

(87) International publication number:
**WO 2016/171975 (27.10.2016 Gazette 2016/43)**

(54) **COMPOSITIONS AND METHODS FOR COPOLYMER SOLUTIONS WITH HIGH WEIGHT PERCENT COPOLYMERS**

ZUSAMMENSETZUNGEN UND VERFAHREN FÜR COPOLYMERLÖSUNGEN MIT COPOLYMER MIT HOHEM GEWICHTSPROZENT

COMPOSITIONS ET PROCÉDÉS POUR DES SOLUTIONS DE COPOLYMÈRES PRÉSENTANT UN POURCENTAGE PONDÉRAL ÉLEVÉ DE COPOLYMÈRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.04.2015 US 201562151877 P**

(43) Date of publication of application:
**28.02.2018 Bulletin 2018/09**

(73) Proprietor: **Cardiac Pacemakers, Inc.**
**St. Paul, Minnesota 55112 (US)**

(72) Inventors:
• **DELANEY, JR., Joseph T.**
**Minneapolis, Minnesota 55418 (US)**

• **WULFMAN, David R.**
**Minneapolis, Minnesota 55405 (US)**
• **AREMU, Adeniyi**
**Brooklyn Park, Minnesota 55444 (US)**
• **ADENUSI, Adegbola O.**
**Burnsville, Minnesota 55306 (US)**

(74) Representative: **Pfenning, Meinig & Partner mbB**
**Patent- und Rechtsanwälte**
**Joachimsthaler Straße 10-12**
**10719 Berlin (DE)**

(56) References cited:
**WO-A1-2011/022583    US-A1- 2010 023 104**

**Description**

TECHNICAL FIELD

The present invention relates to solutions and methods for making solutions of polymers suitable for solvent-based polymer processing. More specifically, the invention relates solutions containing a block copolymer and methods for making solutions including a block copolymer.

BACKGROUND

Polymeric materials are widely used in the field of implantable medical devices. For example, polymeric materials such as silicone rubber, polyurethane, and fluoropolymers are used as coating and/or insulating materials for medical leads, stents, and other devices.

Incorporating polymeric materials into implantable medical devices may be done by a variety of methods, depending on the specific application. In some applications, for example, for implantable lead bodies, the polymeric material may be extruded at a temperature sufficient to cause the block copolymer to flow, but not high enough to cause the polymeric material to break down. That is, the material that forms the lead after the extrusion and cooling has largely the same structure as the original polymeric material.

In other applications, it may be desirable to employ solvent-based processing to incorporate a polymeric material into an implantable medical device. Solvent-based processing includes electrospraying, electrospinning, spray coating, dip coating, and force spinning. Essential to all solvent-based processing of polymeric materials is the ability to bring the polymeric material into solution while retaining the basic structure of the polymeric material. In some cases, the very properties of strength and chemical resistance that characterize some polymeric materials also make it difficult to form solutions of the polymeric materials in which the polymeric material structure remains largely intact. In some cases, solutions of polymeric materials may be limited to only a few weight percent of the polymeric material (e.g. about 1-2 wt. %). Solutions having such low weight percent of polymeric material dissolved may not be economically useful in some solvent-base processing, and may not work at all in other solvent-based processing. In still other cases, it may be possible to bring a polymeric material into solution at adequate concentrations, but the solvent may be so acutely hazardous that its use is undesirable.

SUMMARY

In Example 1, a method of making a solution including a block copolymer includes dissolving a block copolymer in a solvent mixture to form a solution, the solvent mixture including at least two solvent components. A solubility of the block copolymer in the solvent mixture is at least about 7 wt. % at about 64 °C. The solvent mixture is non-reactive with the block copolymer. A maximum solubility of the block copolymer in a single solvent solution consisting of any one of the at least two solvent components and the block copolymer is not greater than about 1 wt. % at about 64 °C. The block copolymer includes polyisobutylene segments and polyurethane segments.

In Example 2, the method of Example 1, wherein a Hansen Solubility Parameter Distance between the block copolymer and the solvent mixture is less than about 2 MPa$^{0.5}$.

In Example 3, the method of any of Examples 1-2, wherein the solvent mixture consists of two solvent components, a first solvent component selected from the group consisting of acetophenone, benzaldehyde, cyclohexanone, and ethyl cinnamate; and a second solvent component selected from the group consisting of aromatic hydrocarbons, cyclohexane, ethyl benzene, Solvesso™ 100, Solvesso™ 150, toluene, and xylene.

In Example 4, the method of any of Examples 1-3, wherein the first solvent component is cyclohexanone, and the second solvent component is toluene.

In Example 5, the method of any of Examples 1-2, wherein the solvent mixture consists of three solvent components, a first solvent component selected from the group consisting of toluene and Solvesso™ 150, a second solvent component selected from the group consisting of phenyl acetonitrile, caprolactone (epsilon), benzaldehyde, and butyl benzoate; and a third solvent component selected from the group consisting of benzyl acetate, tetrahydrofuran, 2,6-dimethylpyridine, benzyl benzoate, and safrole.

In Example 6, the method of Example 1, wherein the solvent mixture includes at least three solvent components, and at least one of the solvent components has a Hansen polar solubility parameter of at least about 5.1 MPa$^{0.5}$, and a Hansen hydrogen bonding parameter of at least about 3.8 MPa$^{0.5}$.

In Example 7, the method of any of Examples 1-6, wherein the solubility of the block copolymer in the solvent mixture is at least about 20 wt. % at about 64 °C.

In Example 8, a method for making an implantable medical device including a block copolymer layer includes formulating a block copolymer solution according to any of claims 1-7, depositing the block copolymer solution onto the implantable medical device; and drying the implantable medical device and evaporating the solvent mixture to leave behind the block copolymer layer.

In Example 9, the method of Example 8, wherein depositing includes at least one of electrospinning and electrospraying the block copolymer solution onto the implantable medical device.

In Example 10, a composition including a first solvent, a second solvent, and a block copolymer. The block copolymer includes polyisobutylene segments and polyurethane segments in solution with the first solvent and the second solvent in an amount no less than about 7 wt. % at about 64 °C. A maximum solubility of the block copolymer in a solution consisting of the first solvent and the block copolymer is not greater than about 1 wt. % at about 64 °C. A maximum solubility of the block copolymer in a solution consisting of the second solvent and the block copolymer is not greater than about 1 wt. % at about 64 °C.

In Example 11, the composition of Example 10, wherein the first solvent is selected from the group consisting of acetophenone, benzaldehyde, cyclohexanone, and ethyl cinnamate; and a second solvent is selected from the group consisting of aromatic hydrocarbons, cyclohexane, ethyl benzene, Solvesso™ 100, Solvesso™ 150, toluene, and xylene.

In Example 12, the composition of any of Examples 10-11, wherein the first solvent is cyclohexanone, and the second solvent is toluene.

In Example 13, the composition of any of Examples 10-12, further including a third solvent, wherein at least one of the solvents has a polar Hansen Solubility Parameter of at least about 5.6 $MPa^{0.5}$ and at least one of the solvents has a hydrogen bonding Hansen Solubility Parameter of at least about 4.6 $MPa^{0.5}$, and a maximum solubility of the block copolymer in a solution consisting of the third solvent and the block copolymer is not greater than about 1 wt. % at about 64 °C.

In Example 14, the composition of Example 10, further including a third solvent, wherein the first solvent is selected from the group consisting of toluene and Solvesso™ 150, a second solvent is selected from the group consisting of phenyl acetonitrile, caprolactone (epsilon), benzaldehyde, and butyl benzoate; and the third solvent is selected from the group consisting of benzyl acetate, tetrahydrofuran, 2,6-dimethylpyridine, benzyl benzoate, and safrole, wherein a maximum solubility of the block copolymer in a solution consisting of the third solvent and the block copolymer is not greater than about 1 wt. % at about 64 °C.

In Example 15, the composition of any of Examples 10-12, wherein the solubility of the block copolymer in the first solvent and the second solvent together is at least about 20 wt. % at about 64 °C.

In Example 16, a method of making a solution including a block copolymer includes dissolving a block copolymer in a solvent mixture to form a solution, the solvent mixture including at least two solvent components. A solubility of the block copolymer in the solvent mixture is at least about 7 wt. % at about 64 °C. The solvent mixture is non-reactive with the block copolymer. A maximum solubility of the block copolymer in a single solvent solution consisting of any one of the at least two solvent components and the block copolymer is not greater than about 1 wt. % at about 64 °C. The block copolymer includes polyisobutylene segments and polyurethane segments.

In Example 17, the method of Example 16, wherein a Hansen Solubility Parameter Distance between the block copolymer and the solvent mixture is less than about 2 $MPa^{0.5}$.

In Example 18, the method of any of Examples 16-17, wherein the solvent mixture consists of two solvent components, a first solvent component selected from the group consisting of acetophenone, benzaldehyde, cyclohexanone, and ethyl cinnamate; and a second solvent component selected from the group consisting of aromatic hydrocarbons, cyclohexane, ethyl benzene, Solvesso™ 100, Solvesso™ 150, toluene, and xylene.

In Example 19, the method of any of claims 16-18, wherein the first solvent component is cyclohexanone, and the second solvent component is toluene.

In Example 20, the method of any of claims 16-17, wherein the solvent mixture consists of three solvent components, a first solvent component selected from the group consisting of toluene and Solvesso™ 150, a second solvent component selected from the group consisting of phenyl acetonitrile, caprolactone (epsilon), benzaldehyde, and butyl benzoate; and a third solvent component selected from the group consisting of benzyl acetate, tetrahydrofuran, 2,6-dimethylpyridine, benzyl benzoate, and safrole.

In Example 21, the method of any of claims 16-17, wherein the solvent mixture includes at least three solvent components, and at least one of the solvent components has a polar Hansen Solubility Parameter of at least about 5.6 $MPa^{0.5}$ and at least one of the solvent components has a hydrogen bonding Hansen Solubility Parameter of at least about 4.6 $MPa^{0.5}$

In Example 22, the method of any of claims 16-21, wherein the solubility of the block copolymer in the solvent mixture is at least about 20 wt. % at about 64 °C.

In Example 23, a composition including a first solvent, a second solvent, and a block copolymer. The block copolymer includes polyisobutylene segments and polyurethane segments in solution with the first solvent and the second solvent in an amount no less than about 7 wt. % at about 64 °C. A maximum solubility of the block copolymer in a solution consisting of the first solvent and the block copolymer is not greater than about 1 wt. % at about 64 °C. A maximum solubility of the block copolymer in a solution consisting of the second solvent and the block copolymer is not greater than about 1 wt. % at about 64 °C.

In Example 24, the composition of Example 23, wherein the first solvent is selected from the group consisting of acetophenone, benzaldehyde, cyclohexanone, and ethyl cinnamate; and a second solvent selected from the group consisting of aromatic hydrocarbons, cyclohexane, ethyl benzene, Solvesso™ 100, Solvesso™ 150, toluene, and xylene.

In Example 25, the composition of any of Examples 23-24, wherein the first solvent is cyclohexanone, and the second solvent is toluene.

In Example 26, the composition of Example 23, further including a third solvent, wherein at least one of the solvents has a polar Hansen Solubility Parameter of at least about 5.6 MPa$^{0.5}$ and at least one of the solvents has a hydrogen bonding Hansen Solubility Parameter of at least about 4.6 MPa$^{0.5}$, and a maximum solubility of the block copolymer in a solution consisting of the third solvent and the block copolymer is not greater than about 1 wt. % at about 64 °C.

In Example 27, the composition of Example 23, wherein the first solvent is selected from the group consisting of toluene and Solvesso™ 150, the second solvent is selected from the group consisting of phenyl acetonitrile, caprolactone (epsilon), benzaldehyde, and butyl benzoate; and the third solvent is selected from the group consisting of benzyl acetate, tetrahydrofuran, 2,6-dimethylpyridine, benzyl benzoate, and safrole, wherein a maximum solubility of the block copolymer in a solution consisting of the third solvent and the block copolymer is not greater than about 1 wt. % at about 64 °C.

In Example 28, the composition of any of examples 23-25, wherein the solubility of the block copolymer in the first solvent and the second solvent together is at least about 20 wt. % at about 64 °C.

In Example 29, a method for making an implantable medical device including a block copolymer layer includes formulating a block copolymer solution including a block copolymer, depositing the block copolymer solution onto the implantable medical device; and drying the implantable medical device and evaporating the solvent mixture to leave behind the block copolymer layer. The block copolymer solution is at a concentration of at least about 7 wt. % at about 64 °C. Formulating the block copolymer solution includes dissolving a block copolymer in a solvent mixture to form the solution, the solvent mixture including at least two solvent components. A solubility of the block copolymer in the solvent mixture is at least about 7 wt. % at about 64 °C. The solvent mixture is non-reactive with the block copolymer. A maximum solubility of the block copolymer in a single solvent solution consisting of any one of the at least two solvent components and the block copolymer is not greater than about 1 wt. % at about 64 °C. The block copolymer includes polyisobutylene segments and polyurethane segments.

In Example 30, the method of Example 29, wherein the solvent mixture consists of two solvent components, a first solvent component selected from the group consisting of acetophenone, benzaldehyde, cyclohexanone, and ethyl cinnamate; and a second solvent component selected from the group consisting of aromatic hydrocarbons, cyclohexane, ethyl benzene, Solvesso™ 100, Solvesso™ 150, toluene, and xylene.

In Example 31, the method of any of Examples 29-30, wherein the first solvent component is cyclohexanone, and the second solvent component is toluene.

In Example 32, the method of Example 29, wherein the solvent mixture consists of three solvent components, a first solvent component selected from the group consisting of toluene and Solvesso™ 150, a second solvent component selected from the group consisting of phenyl acetonitrile, caprolactone (epsilon), benzaldehyde, and butyl benzoate; and a third solvent component selected from the group consisting of benzyl acetate, tetrahydrofuran, 2,6-dimethylpyridine, benzyl benzoate, and safrole.

In Example 33, the method of any of claims 29-32, wherein depositing includes at least one of solvent casting, spray coating, or dip coating the block copolymer solution onto the implantable medical device.

In Example 34, the method of claim 29, wherein the solvent mixture includes at least three solvent components, and at least one of the solvent components has a polar Hansen Solubility Parameter of at least about 5.6 MPa$^{0.5}$ and at least one of the solvent components has a hydrogen bonding Hansen Solubility Parameter of at least about 4.6 MPa$^{0.5}$.

In Example 35, the method of any of claims 29-32 and 34, wherein depositing includes at least one of electrospinning and electrospraying the block copolymer solution onto the implantable medical device.

While multiple embodiments are disclosed, still other embodiments of the present invention will become apparent to those skilled in the art from the following detailed description, which shows and describes illustrative embodiments of the invention. Accordingly, the drawings and detailed description are to be regarded as illustrative in nature and not restrictive.

DETAILED DESCRIPTION

Block copolymers are polymeric materials made of alternating sections of polymerized monomers. Block copolymers may take on a number of configurations, which may be selected, for example, from linear, cyclic and branched configurations, among others. Branched configurations include star-shaped configurations (e.g., configurations in which three or more chains emanate from a single branch point), comb configurations (e.g., configurations having a main chain and a plurality of side chains, also referred to as "graft" configurations), dendritic configurations (e.g., arborescent and hyperbranched polymers), and so forth.

Some block copolymers have properties that are highly desirable for use with implantable medical devices. For example, some block copolymers are known to be strong, durable, oxidation resistant, chemically resistant, and extremely biostable. Such block copolymers are typically difficult to dissolve. Examples of block copolymers suitable for implantable medical devices may include poly(ethylene-co-vinyl acetate), poly(vinylidene fluoride-co-hexafluoropropylene), poly(isobutylene-co-styrene), poly(isobutylene-urethane), and poly(styrene-b-isobutylene-b-styrene). Block copolymers are also tunable, in that their properties can be varied as desired by altering the ratios of the constituent polymerized monomers.

In some embodiments, a solution including a block copolymer may be made by dissolving the block copolymer in a solvent mixture including at least two solvent components or at least three solvent components. Individual solvent components may be homogeneous organic solvents, for example, cyclohexanone or toluene. Together, the solvent components of the solvent mixture dissolve the block copolymer. That is, the block copolymer is dissolved in the solvent mixture such that it is no longer in solid form and will not settle out. The block copolymer is also dissolved such that it retains its basic structure. That is, the dissolved polymer is made of alternating sections of the polymerized monomers of the block copolymer, and less than about 0.1 wt. % of the dissolved polymer is in the form of the monomers or homopolymers.

In some embodiments, the solubility of the block copolymer in the solvent mixture may be at least as great as about 7 wt. %, about 8 wt. %, about 10 wt. %, about 15 wt. %, about 20 wt. %, about 25 wt. %, or about 30 wt. %, or may be no greater than about 40 wt. %, about 50 wt. %, about 60 wt. %, or about 70 wt. %., or may be present within any range defined between any pair of the foregoing values. For example, in some embodiments, the solubility of the block copolymer in the solvent mixture may be in an amount from about 7 wt. % to about 70 wt. %, from about 8 wt. % to about 60 wt. %, from about 10% to about 50%, or from about 20 wt. % to about 40 wt. %. All solubility values described herein are at a temperature of about 64 °C.

Surprisingly, in some embodiments, the solubility of the block copolymer in the solvent mixture may be high as described herein (e.g., up to 70 wt. %), while a maximum solubility of the block copolymer in a single solvent solution (e.g., a solvent mixture comprising a single solvent) of each of the solvents of the solvent mixture may be comparatively low. For example, the maximum solubility of the block copolymer in a single solvent solution may be no greater than about 1 wt. %. Said another way, the maximum solubility of the block copolymer in a plurality of single solvent solutions may be no greater than about 1 wt. % wherein each of the plurality of single solvent solutions consists of any one of the solvent components of the solvent mixture and the block copolymer. In some embodiments, the maximum solubility of the block copolymer in a single solvent solution consisting of any one of the solvent components of the solvent mixture and the block copolymer may be no greater than about 0.5 wt. %, no greater than about 0.2 wt. %, no greater than about 0.1 wt. %, or no greater than about 0.05 wt. %. In some embodiments, the block copolymer may be substantially insoluble in a single solvent solution consisting of any one of the solvent components of the solvent mixture. All maximum solubility values described herein are at a temperature of about 64 °C.

Hansen Solubility Parameters (HSPs) may be used to predict whether a material may dissolve in another. HSPs consist of three parameters representing forces acting between molecules of a substance: dispersion forces, polar intermolecular forces, and hydrogen bonding forces (see Charles M. Hansen, Hansen Solubility Parameters: A User's Handbook (CRC Press, 2d ed. 2007)). The three HSPs define a three-dimensional Hansen space. The three HSPs of a material are coordinates in the Hansen space. Thus, the HSPs of a material, such as a solvent or polymer, determine relative position of the material in the Hansen space. The HSPs of a solvent mixture are a volume-weighted combination of the HSPs of the individual component solvents making up the solvent mixture. Thus, a solvent mixture also has a relative position in Hansen space. A Hansen Solubility Parameter Distance (Ra) is a distance in Hansen space between any two materials, such as a solvent mixture, a solvent component of a solvent mixture, or a polymer. The Ra may be determined from Equation 1 below:

$$Ra = \sqrt{4(\delta_{d2} - \delta_{d1})^2 + (\delta_{p2} - \delta_{p1})^2 + (\delta_{h2} - \delta_{h1})^2} \qquad \textbf{Eq. 1}$$

where $\delta_{d1}$, $\delta_{p1}$, and $\delta_{h1}$ are the dispersion, polar, and hydrogen bonding HSPs, respectively, of one of the two of a solvent mixture, a solvent component of a solvent mixture, or a polymer; and $\delta_{d2}$, $\delta_{p2}$, and $\delta_{h2}$ are the dispersion, polar and hydrogen bonding HSPs of the other of the two of a solvent mixture, a solvent component of a solvent mixture, or a polymer. The values of the HSPs for a particular solvent component or polymer may be determined empirically or may be found in published tables.

An interaction radius ($R_0$) may be determined for a material to be dissolved. $R_0$ is a distance in Hansen space within which any solvent component (or solvent mixture) is likely to form a solution with the material. The ratio of Ra to $R_0$ is known as a Relative Energy Difference (RED). Thus, for a material and a solvent component (or solvent mixture) having RED value greater than 1, the material and solvent component (or solvent mixture) may not be sufficiently similar in their HSPs to form a solution. Conversely, for a material and a solvent (or solvent mixture) having RED value less than 1, the material and solvent component (or solvent mixture) may be sufficiently similar in their HSPs that the material is likely to form a solution with the solvent component (or solvent mixture). The RED may predict whether a material is likely to form a solution with the solvent component (or solvent mixture), but it is not always accurate. The RED does not predict a maximum weight percent of material that will form a solution with the solvent component (or solvent mixture).

In some embodiments, the RED for a polymer and a solvent mixture may be less than about 1, and each of the REDs for the polymer and each of the solvent components of the solvent mixture individually may be greater than about 1. In such embodiments, the RED suggests that each of the solvent components individually is not a suitable solvent for the polymer, but that the solvent mixture may be a suitable solvent for the polymer.

In some embodiments, the solvent mixture may include at least three solvent components and at least one of the solvent components may have a polar HSP of at least about 5.6 $MPa^{0.5}$ and at least one of the solvent components may have a hydrogen bonding HSP of at least about 4.6 $MPa^{0.5}$. Including solvent components having a polar HSP of at least about 5.6 $MPa^{0.5}$ and a hydrogen bonding HSP of at least about 4.6 $MPa^{0.5}$ may improve the efficiency by which the solution including the block copolymer may be employed in some solvent-based processing such as, for example, electrospinning or electrospraying.

In some embodiments, a solution including a block copolymer may be made by dissolving a poly(isobutylene-urethane) block copolymer (PIB-PUR) including polyisobutylene segments and polyurethane segments in a solvent mixture. The PIB-PUR may be synthesized as described in U.S. Patent No. 8,324,290 entitled "Polyisobutylene Urethane, Urea and Urethane/Urea Copolymers and Medical Devices Containing the Same," incorporated herein by reference in its entirety.

In some embodiments, the solvent mixture may include solvent components in which the solubility of the PIB-PUR in the solvent mixture may be at least as great as great as about 7 wt. %, about 8 wt. %, about 10 wt. %, about 15 wt. %, about 20 wt. %, about 25 wt. %, or about 30 wt. %, or may be no greater than about 40 wt. %, about 50 wt. %, about 60 wt. %, or about 70 wt. %., or may be present within any range defined between any pair of the foregoing values. For example, in some embodiments, the solubility of the block copolymer in the solvent mixture may be in an amount from about 7 wt. % to about 70 wt. %, from about 8 wt. % to about 60 wt. %, from about 10% to about 50%, or from about 20 wt. % to about 40 wt. %. The solubilities are determined at about 64 °C.

In some embodiments, the solubility of the PIB-PUR in the solvent mixture may be high as described herein (e.g., about 70 wt. %), while the maximum solubility of the PIB-PUR in a single solvent solution (e.g., a solvent mixture comprising a single solvent) of each of the solvents of the solvent mixture may be comparatively low. For example, the maximum solubility of the PIB-PUR in a single solvent solution may be no greater than about 1 wt. %. Said another way, the maximum solubility of the PIB-PUR in a plurality of single solvent solutions may be no greater than about 1 wt. % wherein each of the plurality of single solvent solutions consists of any one of the solvent components of the solvent mixture and the PIB-PUR. In some embodiments, the maximum solubility of the PIB-PUR in a single solvent solution consisting of any one of the solvent components of the solvent mixture and the PIB-PUR may be no greater than about 0.5 wt. %, no greater than about 0.2 wt. %, no greater than about 0.1 wt. %, or no greater than about 0.05 wt. %. In some embodiments, the PIB-PUR may be substantially insoluble in a single solvent solution consisting of any one of the solvent components of the solvent mixture. The maximum solubilities are determined at a temperature of about 64 °C. Exposure of the PIB-PUR in the solvent mixture to temperatures significantly higher than 64 °C may alter functional or cosmetic properties of the block copolymer.

In some embodiments, a Hansen Solubility Parameter Distance (Ra) between the solvent mixture and the PIB-PUR may be less than about 2.0 $MPa^{0.5}$, less than about 1.8 $MPa^{0.5}$, less than about 1.6 $MPa^{0.5}$, or less than about 1.4 $MPa^{0.5}$. In some embodiments, a Hansen Solubility Parameter Distance (Ra) between any of the solvent components in the solvent mixture and the PIB-PUR may be greater than about 2.5 $MPa^{0.5}$, greater than about 3.0 $MPa^{0.5}$, greater than about 4.0 $MPa^{0.5}$, or greater than about 8.0 $MPa^{0.5}$. A Hansen Solubility Parameter Distance (Ra) between all of the solvent components individually in the solvent mixture and the PIB-PUR may be greater than about 2.5 $MPa^{0.5}$, greater than about 3.0 $MPa^{0.5}$, greater than about 4.0 $MPa^{0.5}$, or greater than about 8.0 $MPa^{0.5}$.

In embodiments of the present disclosure, HSPs for PIB-PUR have been determined empirically. For PIB-PUR the dispersion HSP is about 18.5 $MPa^{0.5}$, the polar HSP is about 5.4 $MPa^{0.5}$, and the hydrogen bonding HSP is about 2.9 $MPa^{0.5}$. HSPs for various solvents may be found in, for example, Charles M. Hansen, Hansen Solubility Parameters: A User's Handbook (CRC Press, 2d ed. 2007) incorporated herein by reference in its entirety.

In some embodiments, the solution including PIB-PUR may be made by dissolving the PIB-PUR in a solvent mixture consisting of two solvent components, a first solvent component and a second solvent component. In some embodiments, the first solvent component may be selected from a group consisting of: acetophenone, benzaldehyde, cyclohexanone, and ethyl cinnamate; and the second solvent component may be selected from a group consisting of aromatic hydrocarbons, cyclohexane, ethyl benzene, Solvesso™ 100, Solvesso™ 150, toluene, and xylene. In some embodiments, the first solvent component may be present in concentrations of greater than or equal to about 50 wt. %, with the balance being the second solvent component. In some embodiments, a weight ratio of the first solvent to the second solvent may be 50% to 50%, 60% to 40%, 70% to 30%, 80% to 20%, or 90% to 10%, or any weight ratio between any of the preceding weight ratios. In other embodiments, the first solvent component may be phenyl acetonitrile, and the second solvent component may be selected from a group consisting of aromatic hydrocarbons, cyclohexane, ethyl benzene, Solvesso™ 100, Solvesso™ 150, toluene, and xylene, wherein the first solvent component may be present in concentrations of less than about 50 wt. %, with the balance being the second solvent component. In some embodiments, a weight ratio of the first solvent to the second solvent may be 49% to 51%, 40% to 60%, 30% to 70%, 20% to 80%, or 10% to 90%, or any weight ratio between any of the preceding weight ratios.

In other embodiments, the solution including PIB-PUR may be made by dissolving the PIB-PUR in a solvent mixture consisting of three solvent components, a first solvent component, a second solvent component, and a third solvent component. In some embodiments, the first solvent component may be selected from a group consisting of toluene and Solvesso™ 150; the second solvent component may be selected from a group consisting of phenyl acetonitrile, caprolactone (epsilon), benzaldehyde, and butyl benzoate; and the third component may be selected from a group consisting of benzyl acetate, tetrahydrofuran, 2,6-dimethylpyridine, benzyl benzoate, and safrole. In some embodiments, the second solvent component may be present in as little as about 20 wt. %, about 25 wt. %, or about 30 wt. %, or may be as great as about 35 wt. %, about 40 wt. %, about 45 wt., about 50 wt. %, or about 55 wt. %, or between any pair of the foregoing values. For example, in some embodiments, the second solvent component may be present in an amount from about 20 wt. % to about 55 wt. %, about 25 wt. % to about 50 wt. %, about 30 wt. % to about 45 wt. %, or about 35 wt. % to about 40 wt. %. In some embodiments, the third solvent component may be present in an amount as little as about 1 wt. %, about 5 wt. %, about 10 wt. %, or about 20 wt. %; with the balance being the first solvent component.

In another embodiment, an implantable medical device including a block copolymer may be made by formulating a block copolymer solution including the block copolymer at a concentration of at least about 7 wt. %, about 8 wt. %, about 10 wt. %, about 15 wt. %, about 20 wt. %, about 25 wt. %, or about 30 wt. %, or no less than about 40 wt. %, about 50 wt. %, about 60 wt. %, or about 70 wt. %., or a concentration within any range defined between any pair of the foregoing values. For example, in some embodiments, the concentration of the block copolymer in the solvent mixture may be in an amount from about 7 wt. % to about 70 wt. %, from about 8 wt. % to about 60 wt. %, from about 10% to about 50%, or from about 20 wt. % to about 40 wt. %. The solubilities are determined at about 64 °C.

Formulating the block copolymer solution may include dissolving the block copolymer in a solvent mixture including at least two solvent components. Together, the solvent components of the solvent mixture dissolve the block copolymer. That is, the block copolymer is dissolved in the solvent mixture such that it is no longer in solid form and will not settle out. The block copolymer is also dissolved such that it retains its basic structure. That is, the dissolved polymer is made of alternating sections of the polymerized monomers of the block copolymer, and less than about 0.1 wt. % of the dissolved polymer is in the form of the monomers or homopolymers.

In some embodiments, drying the implantable medical device includes evaporating the solvent mixture to leave behind the block copolymer layer.

In some embodiments, depositing the block copolymer solution onto the implantable medical device may include at least one of solvent casting, spray coating, or dip coating of the block copolymer solution onto the implantable medical device. In other embodiments, depositing the block copolymer solution onto the implantable medical device may include at least one of electrospinning and electrospraying the block copolymer solution onto the implantable medical device.

In some embodiments, the solvent mixture may include at least three solvent components and at least one of the solvent components may have a polar HSP of at least about 5.6 MPa$^{0.5}$ and a hydrogen bonding HSP of at least about 4.6 MPa$^{0.5}$. Including the solvent component having a polar HSP of at least about 5.6 MPa$^{0.5}$ and a hydrogen bonding HSP of at least about 4.6 MPa$^{0.5}$ may improve the efficiency by which the solution including the block copolymer may be employed in some solvent-based processing such as, for example, electrospinning or electrospraying.

In some embodiments, the block copolymer included in the implantable medical device may include PIB-PUR. The solution including PIB-PUR may be made according to any of the embodiments described above.

In another embodiment, a composition may include a first solvent, a second solvent, and a block copolymer in solution with the first solvent and the second solvent in an amount no less than about 7 wt. %. A maximum solubility of the block copolymer in a solution consisting of the first solvent and the block copolymer may be no greater than about 1 wt. %, and a maximum solubility of the block copolymer in a solution consisting of the second solvent and the block copolymer may be no greater than about 1 wt. %. In some embodiments, the block copolymer may be in solution in an amount no less than about 7 wt. %, about 8 wt. %, about 10 wt. %, about 15 wt. %, about 20 wt. %, about 25 wt. %, or about 30 wt. %, or no greater than about 40 wt. %, about 50 wt. %, about 60 wt. %, or about 70 wt. %, or may be present within any range defined between any pair of the foregoing values. For example, in some embodiments, the solubility of the block copolymer in solution with the first solvent and the second solvent may be in an amount from about 7 wt. % to about 70 wt. %, from about 8 wt. % to about 60 wt. %, from about 10% to about 50%, or from about 20 wt. % to about 40 wt. %. The solubilities are determined at about 64 °C.

In some embodiments, the maximum solubility of the block copolymer in a solution consisting of the first solvent and the block copolymer may be no greater than about 1 wt. %, about 0.5 wt. %, no greater than about 0.2 wt. %, no greater than about 0.1 wt. %, or no greater than about 0.05 wt. %; and the maximum solubility of the block copolymer in a solution consisting of the second solvent and the block copolymer may be no greater than about 1 wt. %, about 0.5 wt. %, no greater than about 0.2 wt. %, no greater than about 0.1 wt. %, or no greater than about 0.05 wt. %. In some embodiments, the block copolymer may be substantially insoluble in the first solvent, and substantially insoluble in the second solvent. The maximum solubilities are determined at about 64 °C.

In some embodiments, the composition may further include a third solvent and at least one of the three solvents may have a polar HSP of at least about 5.6 MPa$^{0.5}$ and at least one of the three solvents may have a hydrogen bonding HSP of at least about 4.6 MPa$^{0.5}$. Including solvents having a polar HSP of at least about 5.6 MPa$^{0.5}$ and a hydrogen bonding HSP of at least about 4.6 MPa$^{0.5}$ may improve the efficiency by which the solution including the block copolymer may be employed in some solvent-based processing such as, for example, electrospinning or electrospraying.

In other embodiments, block copolymer of the composition may include polyisobutylene segments and polyurethane segments (PIB-PUR). In some embodiments, the first solvent of the composition may be selected from a group consisting of : acetophenone, benzaldehyde, cyclohexanone, and ethyl cinnamate; and the second solvent of the composition may be selected from a group consisting of aromatic hydrocarbons, cyclohexane, ethyl benzene, Solvesso™ 100, Solvesso™ 150, toluene, and xylene. In some embodiments, the first solvent may be present in concentrations of greater than or equal to about 50 wt. %, with the balance being the second solvent. In some embodiments, a weight ratio of the first solvent to the second solvent may be 50% to 50%, 60% to 40%, 70% to 30%, 80% to 20%, or 90% to 10%, or any weight ratio between any of the preceding weight ratios. In other embodiments, the first solvent may be phenyl acetonitrile, and the second solvent may be selected from a group consisting of aromatic hydrocarbons, cyclohexane, ethyl benzene, Solvesso™ 100, Solvesso™ 150, toluene, and xylene, in which the first solvent may be present in concentrations of less than about 50 wt. %, with the balance being the second solvent. In some embodiments, a weight ratio of the first solvent to the second solvent may be 49% to 51%, 40% to 60%, 30% to 70%, 20% to 80%, or 10% to 90%, or any weight ratio between any of the preceding weight ratios. In some embodiments, the first solvent may be cyclohexanone, and the second solvent may be toluene.

In other embodiments, the first solvent of the composition may be selected from a group consisting of toluene and Solvesso™ 150; the second solvent of the composition may be selected from a group consisting of phenyl acetonitrile, caprolactone (epsilon), benzaldehyde, and butyl benzoate; and the third solvent of the composition may be selected from a group consisting of benzyl acetate, tetrahydrofuran, 2,6-dimethylpyridine, benzyl benzoate, and safrole. In some embodiments, the second solvent may be present in as little as about 20 wt. %, about 25 wt. %, or about 30 wt. %, or may be as great as about 35 wt. %, about 40 wt. %, about 45 wt., about 50 wt. %, or about 55 wt. %, or between any pair of the foregoing values. For example, in some embodiments, the second solvent may be present in an amount from about 20 wt. % to about 55 wt. %, about 25 wt. % to about 50 wt. %, about 30 wt. % to about 45 wt. %, or about 35 wt. % to about 40 wt. %. In some embodiments, the third solvent may be present in an amount as little as about 1 wt. %, about 5 wt. %, about 10 wt. %, or about 20 wt. %; and the balance being the first solvent.

EXAMPLES

The present invention is more particularly described in the following examples that are intended as illustrations only, since numerous modifications and variations within the scope of the present invention will be apparent to those of skill in the art. Unless otherwise noted, all parts, percentages, and ratios reported in the following examples are on a weight bases, and all reagents used in the examples were obtained, or are available, from the chemical suppliers described below, or may be synthesized by conventional techniques.

**Example 1**

**PIB-PUR in Cyclohexanone/Toluene**

PIB-PUR was synthesized as described in U.S. Patent No. 8,324,290 entitled "Polyisobutylene Urethane, Urea and Urethane/Urea Copolymers and Medical Devices Containing the Same," incorporated herein by reference in its entirety. PIB-PUR formed by this method is produced as a thick film of polymer material. The PIB-PUR was cut or broken apart into polymer chunks having a largest dimension from about 2 millimeters (mm) to about 20 mm.

PIB-PUR was added to cyclohexanone (Sigma-Aldrich, 99.8%, #W390909). The cyclohexanone was stirred and held at a temperature of 64 °C for at least 48 hours to as long as 96 hours. The PIB-PUR was observed to have not dissolved appreciably in the cyclohexanone. PIB-PUR was observed by eye to have swelled slightly when added to the cyclohexanone. The Hansen Solubility Parameter Distance (Ra) between cyclohexanone and PIB-PUR is about 4.0 MPa$^{0.5}$.

PIB-PUR was added to toluene (Sigma-Aldrich, 99.5%, #179418). The toluene was stirred and held at a temperature of 64°C for at least 48 hours to as long as 96 hours. The PIB-PUR was observed to have not dissolved appreciably in the toluene. PIB-PUR was observed by eye to have swelled slightly when added to the toluene. The edges were observed turning transparent while the center remained opaque. The Ra between toluene and PIB-PUR is about 4.2 MPa$^{0.5}$.

Cyclohexanone and toluene were combined in equal volumes to form a solvent mixture. The solvent mixture was stirred and held at a temperature of 64 °C. PIB-PUR was added to the solvent mixture in increasing weight percentages. PIB-PUR at various weight percentages up to 17.8 wt. % completely dissolved in the solvent mixture to form a solution after 24 hours. The Ra between the 50/50 cyclohexanone/toluene solvent mixture and PIB-PUR is about 1.4 MPa$^{0.5}$.

## Claims

1. A method of making a solution including a block copolymer, the method comprising:
   dissolving a block copolymer in a solvent mixture to form a solution, the solvent mixture including at least two solvent components, wherein a solubility of the block copolymer in the solvent mixture is at least about 7 wt. % at about 64 °C, the solvent mixture is non-reactive with the block copolymer, and a maximum solubility of the block copolymer in a single solvent solution consisting of any one of the at least two solvent components and the block copolymer is not greater than about 1 wt. % at about 64 °C, wherein the block copolymer includes polyisobutylene segments and polyurethane segments.

2. The method of claim 1, wherein a Hansen Solubility Parameter Distance between the block copolymer and the solvent mixture is less than about 2 MPa$^{0.5}$.

3. The method of any of claims 1-2, wherein the solvent mixture consists of two solvent components, a first solvent component selected from the group consisting of acetophenone, benzaldehyde, cyclohexanone, and ethyl cinnamate; and a second solvent component selected from the group consisting of aromatic hydrocarbons, cyclohexane, ethyl benzene, toluene, and xylene.

4. The method of claim 3, wherein the first solvent component is cyclohexanone, and the second solvent component is toluene.

5. The method of any of claims 1-2, wherein the solvent mixture consists of three solvent components, a first solvent component selected from the group consisting of toluene and Solvesso™ 150, a second solvent component selected from the group consisting of phenyl acetonitrile, caprolactone (epsilon), benzaldehyde, and butyl benzoate; and a third solvent component selected from the group consisting of benzyl acetate, tetrahydrofuran, 2,6-dimethylpyridine, benzyl benzoate, and safrole.

6. The method of claim 1, wherein the solvent mixture includes at least three solvent components, and at least one of the solvent components has a Hansen polar solubility parameter of at least about 5.1 MPa$^{0.5}$, and a Hansen hydrogen bonding parameter of at least about 3.8 MPa$^{0.5}$.

7. The method of any of claims 1-6, wherein the solubility of the block copolymer in the solvent mixture is at least about 20 wt. % at about 64 °C.

8. A method for making an implantable medical device including a block copolymer layer, the method comprising:

   formulating a block copolymer solution according to any of claims 1-7,
   depositing the block copolymer solution onto the implantable medical device; and
   drying the implantable medical device and evaporating the solvent mixture to leave behind the block copolymer layer.

9.  The method of claim 8, wherein depositing includes at least one of electrospinning and electrospraying the block copolymer solution onto the implantable medical device.

10. A composition comprising:

   a first solvent;
   a second solvent; and
   a block copolymer including polyisobutylene segments and polyurethane segments in solution with the first solvent and the second solvent in an amount no less than about 7 wt. % at about 64 °C,
   wherein a solubility of the block copolymer in a solution consisting of the first solvent and the block copolymer is not greater than about 1 wt. % at about 64 °C, and a solubility of the block copolymer in a solution consisting of the second solvent and the block copolymer is not greater than about 1 wt. % at about 64 °C.

11. The composition of claim 10, wherein the first solvent is selected from the group consisting of acetophenone, benzaldehyde, cyclohexanone, and ethyl cinnamate; and a second solvent selected from the group consisting of aromatic hydrocarbons, cyclohexane, ethyl benzene, Solvesso™ 100, Solvesso™ 150, toluene, and xylene.

12. The composition of any of claims 10-11, wherein the first solvent is cyclohexanone, and the second solvent is toluene.

13. The composition of any of claims 10-12, further including a third solvent, wherein at least one of the solvents has a polar Hansen Solubility Parameter of at least about 5.6 MPa$^{0.5}$ and at least one of the solvents has a hydrogen bonding Hansen Solubility Parameter of at least about 4.6 MPa$^{0.5}$, and the solubility of the block copolymer in a solution consisting of the third solvent and the block copolymer is not greater than about 1 wt. % at about 64 °C.

14. The composition of claim 10, further including a third solvent, wherein the first solvent is selected from the group consisting of toluene and Solvesso™ 150, the second solvent is selected from the group consisting of phenyl acetonitrile, caprolactone (epsilon), benzaldehyde, and butyl benzoate; and the third solvent is selected from the group consisting of benzyl acetate, tetrahydrofuran, 2,6-dimethylpyridine, benzyl benzoate, and safrole, wherein a maximum solubility of the block copolymer in a solution consisting of the third solvent and the block copolymer is not greater than about 1 wt. % at about 64 °C.

15. The composition of any of claims 10-12, wherein the solubility of the block copolymer in the first solvent and the second solvent together is at least about 20 wt. % at about 64 °C.

**Patentansprüche**

1.  Verfahren zur Herstellung einer Lösung, die ein Blockcopolymer enthält, wobei das Verfahren umfasst:
   Auflösen eines Blockcopolymers in einem Lösungsmittelgemisch, um eine Lösung zu bilden, wobei das Lösungsmittelgemisch mindestens zwei Lösungsmittelkomponenten enthält, wobei die Löslichkeit des Blockcopolymers in dem Lösungsmittelgemisch bei etwa 64 °C mindestens etwa 7 Gew.-% beträgt, das Lösungsmittelgemisch mit dem Blockcopolymer nicht reaktiv ist, und eine maximale Löslichkeit des Blockcopolymers in einer einzelnen Lösungsmittellösung, die aus einer der mindestens zwei Lösungsmittelkomponenten und dem Blockcopolymer besteht, bei etwa 64 °C nicht größer als etwa 1 Gew.-% ist, wobei das Blockcopolymer Polyisobutylensegmente und Polyurethansegmente enthält.

2.  Verfahren nach Anspruch 1, wobei ein Hansen-Löslichkeitsparameterabstand zwischen dem Blockcopolymer und dem Lösungsmittelgemisch weniger als etwa 2 MPa$^{0,5}$ beträgt.

3.  Verfahren nach einem der Ansprüche 1 bis 2, wobei das Lösungsmittelgemisch aus zwei Lösungsmittelkomponenten besteht, einer ersten Lösungsmittelkomponente, die ausgewählt ist aus der Gruppe bestehend aus Acetophenon, Benzaldehyd, Cyclohexanon und Ethylcinnamat; und einer zweiten Lösungsmittelkomponente, die ausgewählt ist aus der Gruppe bestehend aus aromatischen Kohlenwasserstoffen, Cyclohexan, Ethylbenzol, Toluol und Xylol.

4.  Verfahren nach Anspruch 3, wobei die erste Lösungsmittelkomponente Cyclohexanon ist und die zweite Lösungsmittelkomponente Toluol ist.

5.  Verfahren nach einem der Ansprüche 1 bis 2, wobei das Lösungsmittelgemisch aus drei Lösungsmittelkomponenten besteht, einer ersten Lösungsmittelkomponente, die ausgewählt ist aus der Gruppe bestehend aus Toluol und Solvesso™ 150, einer zweiten Lösungsmittelkomponente, die ausgewählt ist aus der Gruppe bestehend aus Phenylacetonitril, Caprolacton (Epsilon), Benzaldehyd und Butylbenzoat; und einer dritten Lösungsmittelkomponente, die ausgewählt ist aus der Gruppe bestehend aus Benzylacetat, Tetrahydrofuran, 2,6-Dimethylpyridin, Benzylbenzoat und Safrol.

6. Verfahren nach Anspruch 1, wobei das Lösungsmittelgemisch mindestens drei Lösungsmittelkomponenten enthält und mindestens eine der Lösungsmittelkomponenten einen Hansen-Parameter für die polare Löslichkeit von mindestens etwa 5,1 MPa$^{0,5}$ und einen Hansen-Wasserstoffbindungsparameter von mindestens etwa 3,8 MPa$^{0,5}$ aufweist.

7. Verfahren nach einem der Ansprüche 1 bis 6, wobei die Löslichkeit des Blockcopolymers in dem Lösungsmittelgemisch bei etwa 64 °C mindestens etwa 20 Gew.-% beträgt.

8. Verfahren zur Herstellung einer implantierbaren medizinischen Vorrichtung, die eine Blockcopolymerschicht aufweist, wobei das Verfahren umfasst:

Formulieren einer Blockcopolymerlösung nach einem der Ansprüche 1 bis 7,
Ablagern der Blockcopolymerlösung auf der implantierbaren medizinischen Vorrichtung; und
Trocknen der implantierbaren medizinischen Vorrichtung und Verdampfen der Lösungsmittelmischung, um die Blockcopolymerschicht zurückzulassen.

9. Verfahren nach Anspruch 8, wobei das Abscheiden mindestens eines von Elektrospinnen und Elektrosprühen der Blockcopolymerlösung auf die implantierbare medizinische Vorrichtung umfasst.

10. Zusammensetzung, enthaltend:

ein erstes Lösungsmittel;
ein zweites Lösungsmittel; und
ein Blockcopolymer mit Polyisobutylensegmenten und
Polyurethansegmenten in Lösung mit dem ersten Lösungsmittel und dem zweiten Lösungsmittel in einer Menge von nicht weniger als etwa 7 Gew.-%. bei etwa 64 °C,
wobei eine Löslichkeit des Blockcopolymers in einer Lösung, die aus dem ersten Lösungsmittel und dem Blockcopolymer besteht, bei etwa 64 °C nicht größer als etwa 1 Gew.-% ist, und die Löslichkeit des Blockcopolymers in einer Lösung, die aus dem zweiten Lösungsmittel und dem Blockcopolymer besteht, bei etwa 64 °C nicht größer als etwa 1 Gew.-% ist.

11. Zusammensetzung nach Anspruch 10, wobei das erste Lösungsmittel ausgewählt ist aus der Gruppe bestehend aus Acetophenon, Benzaldehyd, Cyclohexanon und Ethylcinnamat; und ein zweites Lösungsmittel ausgewählt ist aus der Gruppe bestehend aus aromatischen Kohlenwasserstoffen, Cyclohexan, Ethylbenzol, Solvesso™ 100, Solvesso™ 150, Toluol und Xylol.

12. Zusammensetzung nach einem der Ansprüche 10 bis 11, wobei das erste Lösungsmittel Cyclohexanon ist und das zweite Lösungsmittel Toluol ist.

13. Zusammensetzung nach einem der Ansprüche 10 bis 12, die ferner ein drittes Lösungsmittel enthält, wobei mindestens eines der Lösungsmittel einen polaren Hansen-Löslichkeitsparameter von mindestens etwa 5,6 MPa$^{0,5}$ aufweist und mindestens eines der Lösungsmittel einen Wasserstoffbrückenbindungs-Hansen-Löslichkeitsparameter von mindestens etwa 4,6 MPa$^{0,5}$ aufweist und die Löslichkeit des Blockcopolymers in einer Lösung, die aus dem dritten Lösungsmittel und dem Blockcopolymer besteht, bei etwa 64 °C nicht größer als etwa 1 Gew.-% ist.

14. Zusammensetzung nach Anspruch 10, ferner enthaltend ein drittes Lösungsmittel, wobei das erste Lösungsmittel aus der Gruppe bestehend aus Toluol und Solvesso™ 150 ausgewählt ist, das zweite Lösungsmittel aus der Gruppe bestehend aus Phenylacetonitril, Caprolacton (Epsilon), Benzaldehyd und Butylbenzoat ausgewählt ist; und das dritte Lösungsmittel aus der Gruppe bestehend aus Benzylacetat, Tetrahydrofuran, 2,6-Dimethylpyridin, Benzylbenzoat und Safrol ausgewählt ist, wobei eine maximale Löslichkeit des Blockcopolymers in einer Lösung, die aus dem dritten Lösungsmittel und dem Blockcopolymer besteht, bei etwa 64 °C nicht besteht mehr als etwa 1 Gew.-% beträgt.

**15.** Zusammensetzung nach einem der Ansprüche 10 bis 12, wobei die Löslichkeit des Blockcopolymers in dem ersten Lösungsmittel und dem zweiten Lösungsmittel bei etwa 64 °C zusammen mindestens etwa 20 Gew.-% beträgt.

**Revendications**

**1.** Procédé de fabrication d'une solution qui inclut un copolymère bloc, le procédé comprenant :
la dissolution d'un copolymère bloc dans un mélange de solvants de manière à former une solution, le mélange de solvants incluant au moins deux composants de solvant, dans lequel une solubilité du copolymère bloc dans le mélange de solvants est d'au moins environ 7 % en poids à environ 64 °C, le mélange de solvants est non réactif avec le copolymère bloc, et une solubilité maximum du copolymère bloc dans une solution à solvant unique qui est constituée par l'un quelconque des au moins deux composants de solvant et par le copolymère bloc n'est pas supérieure à environ 1 % en poids à environ 64 °C, dans lequel le copolymère bloc inclut des segments de polyisobutylène et des segments de polyuréthane.

**2.** Procédé selon la revendication 1, dans lequel une distance de paramètres de solubilité de Hansen entre le copolymère bloc et le mélange de solvants est inférieure à environ 2 MPa$^{0,5}$.

**3.** Procédé selon l'une quelconque des revendications 1 et 2, dans lequel le mélange de solvants est constitué par deux composants de solvant, soit un premier composant de solvant qui est sélectionné parmi le groupe qui est constitué par l'acétophénone, le benzaldéhyde, le cyclohexanone et le cinnamate d'éthyle ; et un second composant de solvant qui est sélectionné parmi le groupe qui est constitué par les hydrocarbures aromatiques, le cyclohexane, l'éthylbenzène, le toluène et le xylène.

**4.** Procédé selon la revendication 3, dans lequel le premier composant de solvant est le cyclohexanone, et le second composant de solvant est le toluène.

**5.** Procédé selon l'une quelconque des revendications 1 et 2, dans lequel le mélange de solvants est constitué par trois composants de solvant, soit un premier composant de solvant qui est sélectionné parmi le groupe qui est constitué par le toluène et le Solvesso™ 150 ; un deuxième composant de solvant qui est sélectionné parmi le groupe qui est constitué par le phénylacétonitrile, le caprolactone (epsilon), le benzaldéhyde et le benzoate de butyle ; et un troisième composant de solvant qui est sélectionné parmi le groupe qui est constitué par l'acétate de benzyle, le tétrahydrofurane, la 2,6-diméthylpyridine, le benzoate de benzyle et le safrole.

**6.** Procédé selon la revendication 1, dans lequel le mélange de solvants inclut au moins trois composants de solvant, et au moins l'un des composants de solvant présente un paramètre de solubilité polaire de Hansen d'au moins environ 5,1 MPa$^{0,5}$, et un paramètre de liaison hydrogène de Hansen d'au moins environ 3,8 MPa$^{0,5}$.

**7.** Procédé selon l'une quelconque des revendications 1 à 6, dans lequel la solubilité du copolymère bloc dans le mélange de solvants est d'au moins environ 20 % en poids à environ 64 °C.

**8.** Procédé pour fabriquer un dispositif médical implantable incluant une couche de copolymère bloc, le procédé comprenant :

la formulation d'une solution de copolymère bloc selon l'une quelconque des revendications 1 à 7 ;
le dépôt de la solution de copolymère bloc sur le dispositif médical implantable ; et
le séchage du dispositif médical implantable et l'évaporation du mélange de solvants de manière à laisser derrière la couche de copolymère bloc.

**9.** Procédé selon la revendication 8, dans lequel le dépôt inclut au moins une technique de dépôt prise parmi l'électrofilage et l'électro-pulvérisation de la solution de copolymère bloc sur le dispositif médical implantable.

**10.** Composition comprenant :

un premier solvant ;
un second solvant ; et
un copolymère bloc qui inclut des segments de polyisobutylène et des segments de polyuréthane en solution avec le premier solvant et avec le second solvant selon une quantité non inférieure à environ 7 % en poids à environ 64 °C ; dans laquelle :

une solubilité du copolymère bloc dans une solution qui est constituée par le premier solvant et par le copolymère bloc n'est pas supérieure à environ 1 % en poids à environ 64 °C, et une solubilité du copolymère bloc dans une solution qui est constituée par le second solvant et par le copolymère bloc n'est pas supérieure à environ 1 % en poids à environ 64 °C.

11. Composition selon la revendication 10, dans laquelle le premier solvant est sélectionné parmi le groupe qui est constitué par l'acétophénone, le benzaldéhyde, le cyclohexanone et le cinnamate d'éthyle ; et un second solvant est sélectionné parmi le groupe qui est constitué par les hydrocarbures aromatiques, le cyclohexane, l'éthylbenzène, le Solvesso™ 100, le Solvesso™ 150, le toluène et le xylène.

12. Composition selon l'une quelconque des revendications 10 et 11, dans laquelle le premier solvant est le cyclohexanone, et le second solvant est le toluène.

13. Composition selon l'une quelconque des revendications 10 à 12, incluant en outre un troisième solvant, dans laquelle au moins l'un des solvants présente un paramètre de solubilité polaire de Hansen d'au moins environ 5,6 MPa$^{0,5}$ et au moins l'un des solvants présente un paramètre de solubilité de liaison hydrogène de Hansen d'au moins environ 4,6 MPa$^{0,5}$, et la solubilité du copolymère bloc dans une solution qui est constituée par le troisième solvant et par le copolymère bloc n'est pas supérieure à environ 1 % en poids à environ 64 °C.

14. Composition selon la revendication 10, incluant en outre un troisième solvant, dans laquelle le premier solvant est sélectionné parmi le groupe qui est constitué par le toluène et le Solvesso™ 150 ; le deuxième solvant est sélectionné parmi le groupe qui est constitué par le phénylacétonitrile, le caprolactone (epsilon), le benzaldéhyde et le benzoate de butyle ; et le troisième solvant est sélectionné parmi le groupe qui est constitué par l'acétate de benzyle, le tétrahydrofurane, la 2,6-diméthylpyridine, le benzoate de benzyle et le safrole, dans laquelle une solubilité maximum du copolymère bloc dans une solution qui est constituée par le troisième solvant et par le copolymère bloc n'est pas supérieure à environ 1 % en poids à environ 64 °C.

15. Composition selon l'une quelconque des revendications 10 à 12, dans laquelle la solubilité du copolymère bloc dans le premier solvant et dans le second solvant en association est d'au moins environ 20 % en poids à environ 64 °C.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8324290 B

**Non-patent literature cited in the description**

- **CHARLES M. HANSEN.** Hansen Solubility Parameters: A User's Handbook. CRC Press, 2007